# EUROPEAN PATENT APPLICATION

(11) **EP 1 174 430 A1**
(43) Date of publication of application: **23.01.2002**
(21) Application number: 00115677.7
(22) Date of filing: 20.07.2000
(51) Int. Cl.: C07D 277/66, C07D 263/57

(54) **Process for the preparation of heterocycles by using C1-building blocks**

(71) Applicant: Universität Konstanz, 78434 Konstanz (DE)
(72) Inventor: Daltrozzo, Ewald, Dr., 78467 Konstanz (DE); Reiss, Alexander, Dr., 88699 Frickingen (DE)
(74) Representative: Müller-Boré & Partner Patentanwälte

(57) **Abstract**

Process for preparing heterocycles having the structural units 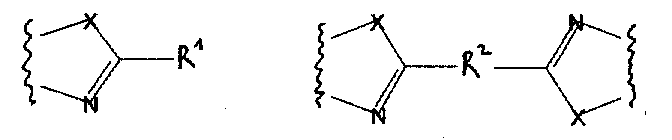 by recation carboxylic halides R¹-COY or YCO-R²-COY, wherein Y is halogen, with compounds having at least the substituents -XH and -NH₂.

## Description

The present invention relates to a process for producing heterocycles having at least two heteroatoms at least one of which being a nitrogen atom by reacting specific carboxylic acid halogenides as a C₁-building block with specific bifunctional compounds.

A typical known process for producing benzofused 5-membered heterocycles having two heteroatoms, for example compounds having the formula (A), starts from o-disubstituted aromatic compounds and carboxylic acids or esters thereof (Hein, J. Am. Chem. Soc. 79 (1957) 427; Hutchinson, Ian, J. Med. Chem. 42, 3 (1999), 381). 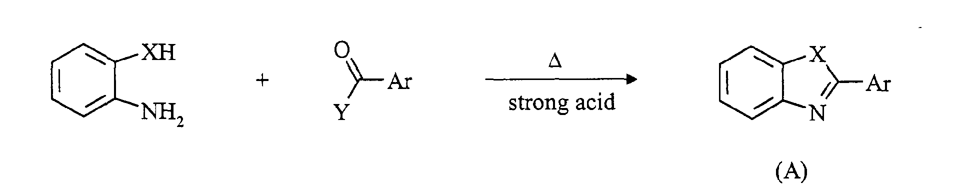 wherein
X is O, S or NR⁵ (R⁵ is H, alkyl or aryl);
Y is OR⁵ (R⁵ is H, alkyl or aryl); and
Ar is aryl.

The above reaction requires the use of strong acids, e.g. polyphosphoric acid or pyrophosphoric acid, and only takes place when drastic reaction conditions are applied, i.e. high reaction temperature and long reaction time. Further drawbacks of this process are on the one hand complex work-up and purification procedures and on the other hand poor yields of the heterocycles. Moreover, such a harsh process is not applicable, if thermolabile or polyfunctional starting materials are used.

In the prior art, there is disclosed the synthesis of benzothiazoles (B) by reacting an aromatic aldehyde with o-mercapto aniline (Bogert, Stull, J. Am. Chem. Soc. 47 (1925)3080) 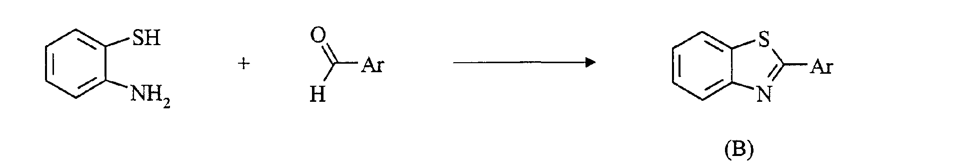 wherein Ar is aryl. An obvious drawback of this reaction, however, is the limited accessibility of aldehydes required and the restriction to only one aromatic heterocycle, namely benzothiazole (B).

Thus, the technical problem underlying the present invention is to overcome the above mentioned drawbacks of the processes known in the prior art and to provide an improved process for producing heterocycles by the use of readily accessible C₁-building blocks.

The solution to the above mentioned technical problem is achieved by the embodiments as defined in the claims. In particular, the present invention provides a process for producing heterocycles having at least two heteroatoms at least one of which being a nitrogen atom and having the structural units (1) or (2): 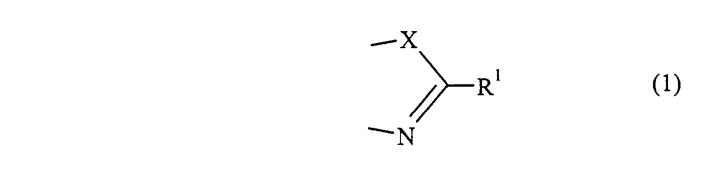 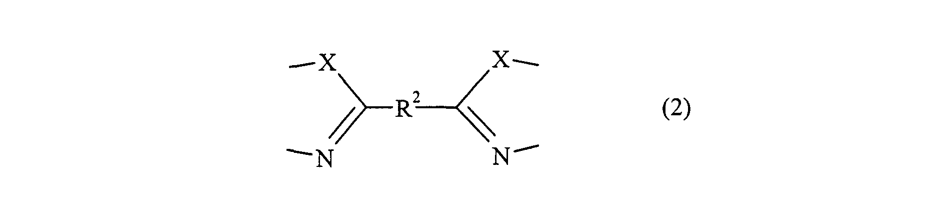 wherein X and N are linked via a substituted or unsubstituted C₂-C₄ alkylene group, preferably ethylene, propylene or butylene, via the 1,2-, 1,3- or 1,4-position of a substituted or unsubstituted (C₅-C₇) cycloaliphatic group, preferably cyclopentane-1,2-diyl or cyclohexane-1,2-diyl, via the 1,2-position of the double bond of a substituted or unsubstituted alkenylene group, preferably ethenylene, via the 1,2-position of a substituted or unsubstituted phenylene group, preferably 1,2-phenylene, wherein the phenylene group can be coupled by a C-C single bond to form a biphenylene group, preferably 3,3',4,4'-biphenylene, having the structural unit (1) on both phenylic units, via the 1,8-position of a substituted or unsubstituted naphthylene group, preferably 1,8-naphthylene, or via other substituted or unsubstituted 5- or 6-membered aromatic or cycloaliphatic rings having optionally one or more heteroatoms, or benzofused derivatives thereof, wherein compounds having the structural unit (2) may be symmetrically or unsymmetrically; and
wherein
X represents O, S, 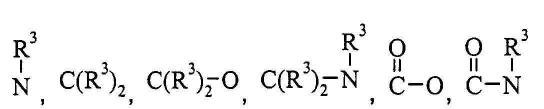 with R³ being each independently selected from hydrogen, a substituted or unsubstituted alkyl group, preferably methyl, ethyl, propyl, or hexyl, a substituted or unsubstituted aryl group, preferably phenyl, or a substituted or unsubstituted heterocyclic group;
R¹ is selected from a carboxyalkyl group, preferably a carboxymethyl or carboxyethyl group, a substituted or unsubstituted alkyl group, preferably methyl, a substituted or unsubstituted aryl group, preferably phenyl, 2-hydroxyphenyl, 2-aminophenyl, 2-methylaminophenyl, 2-dimethylaminophenyl, 2-nitrophenyl, or 2-chlorophenyl, a substituted or unsubstituted heterocyclic group, preferably coumaryl, flavyl, 2-pyridyl or 2-piperidyl, or derivatives thereof;
R² is selected from a substituted or unsubstituted alkylene group, preferably methylene, ethylene, propylene, butylene, pentylene or hexylene, a substituted or unsubstituted alkenylene group, preferably cis-ethenylene, trans-ethenylene or trans,trans-buta-1,3-dienylene, a substituted or unsubstituted alkynylene group, preferably ethynylene or butynylene, a substituted or unsubstituted arylene group, preferably 1,2-phenylene, 1,3-phenylene, 1,4-phenylene, 4,4'-biphenylene, 1,5-naphthylene or 9,10-anthrylene, or a substituted or unsubstituted bivalent heterocyclic group, preferably 2,5-pyrazinyl, 3,6-pyridazinyl, 2,4-pyrimidinyl, 2,5-pyrimidinyl or 2,6- pyrimidinyl;
the process comprising the step of reacting a carboxylic acid halogenide having the general formulae (3) or (4): 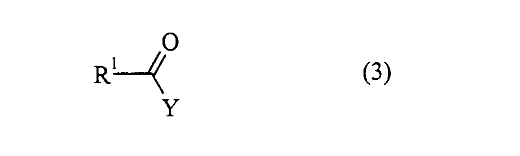 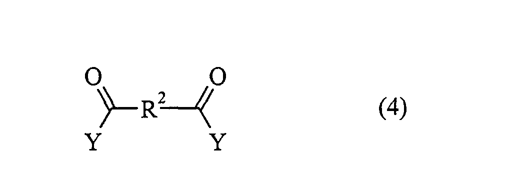 wherein R¹ and R² are as defined above and Y represents a halogen atom, with bifunctional compounds having at least two substituents -XH and -NH₂ at a temperature in the range of -50°C to 50°C in an aprotic solvent, wherein X is as defined above and X and N are linked via a substituted or unsubstituted C₂-C₄ alkylene group, preferably ethylene, propylene or butylene, via the 1,2-, 1,3- or 1,4-position of a substituted or unsubstituted cycloaliphatic C₅-C₇ group, preferably cyclopentane-1,2-diyl or cyclohexane-1,2-diyl group, via the 1,2-position of the double bond of a substituted or unsubstituted alkenylene group, preferably ethenylene, via the 1,2-position of a substituted or unsubstituted phenylene group, preferably 1,2-phenylene, wherein the phenylene group can be coupled by a C-C single bond to form a biphenylene group, preferably 3,3',4,4'-biphenylene, via the 1,8-position of a substituted or unsubstituted naphthylene group, preferably 1,8-naphthylene, or via other substituted or unsubstituted 5- or 6-membered aromatic or cycloaliphatic rings having optionally one or more heteroatoms, or benzofused derivatives thereof.

The process according to the present invention can advantageously be carried out under extremely mild temperatures. The reaction milieu of the process according to the present invention is slightly alkaline. Advantageously, the reaction time of the process is significantly shortened and the yields of the heterocycles obtainable by the process according to the present invention are excellent (see Table 1 below). Furthermore, the use of strong acids can be avoided during the process according to the present invention. To the contrary, conventional processes known in the prior art require high reaction temperatures, a long reaction time as well as a strong acidic reaction milieu. Furthermore, the yields of the products strongly depend on the starting materials. The following examples as shown in Table 1 clearly demonstrate the superior yields being achievable by the process of the present invention, compared with the conventional carboxylic acid process known in the prior art.

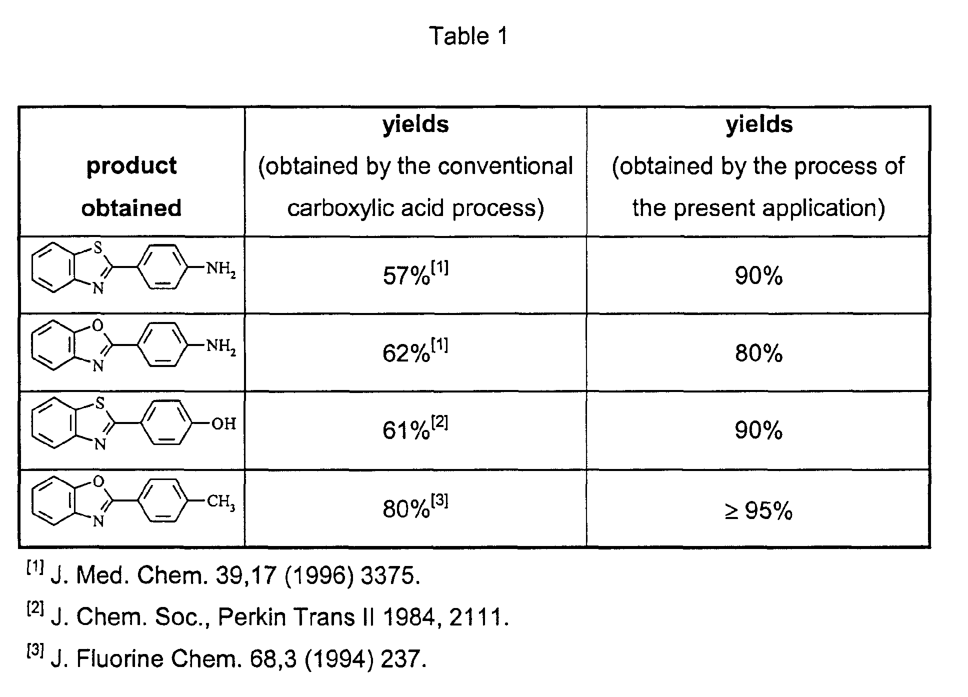

The compounds achievable by the process according to the present invention are of considerable interest, since they can be used as fluorescence dyes or laser dyes and represent starting materials for the synthesis of a large variety of metal chelates. Moreover, these compounds are of great importance in pharmacology and bioanalysis.

For example, compounds being achievable by the process according to the present invention, can be represented by the following structures:

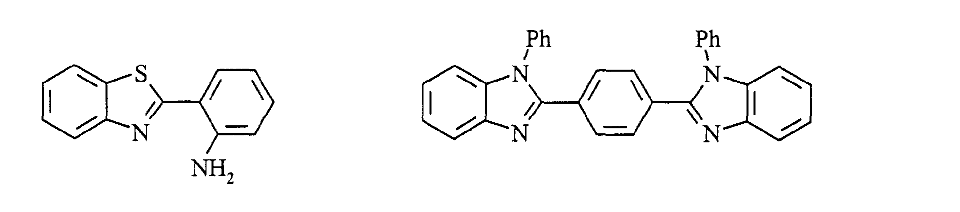

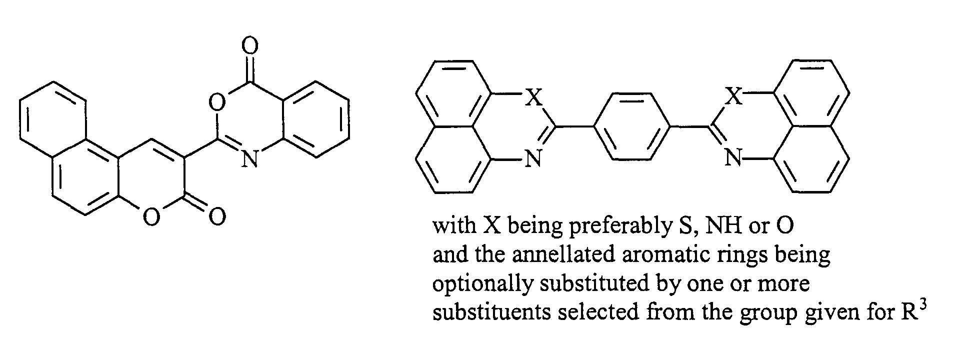

Particularly, if the process of the present invention is carried out by the use of a carboxylic acid chloride (X = Cl in the general formulae (3) or (4)), the isolated yields of the respective heterocycles thus obtained are very good (80 to ≥ 95%, see Table 1), the reaction time is short and the reaction temperature to be employed can be low. Moreover, carboxylic acid chlorides are readily accessible, e.g. by reacting a carboxylic acid with thionyl chloride. Further suitable carboxylic acid halogenides are carboxylic acid bromides and iodides.

According to a preferred embodiment of the present invention X represents an oxygen or sulphur atom or a group NR³ with R³ being as defined above. Preferably, R³ is selected from methyl, ethyl, propyl, isopropyl, butyl, hexyl, dodecyl, phenyl, or naphthyl.

In particular, R¹ in the general formula (3) can represent a coumaryl group or a benzofused coumaryl group or derivatives thereof, or a substituted aryl or heteroaryl group comprising one or more substituents selected from a hydroxy group, an alkoxy group, preferably methoxy, an amino group, an alkylamino group, preferably methylamino, a dialkylamino group, preferably dimethylamino, a cyano group, a carboxyl group or a nitro group.

According to a further preferred embodiment of the process of the present invention, the compounds of the general formula (4) are: 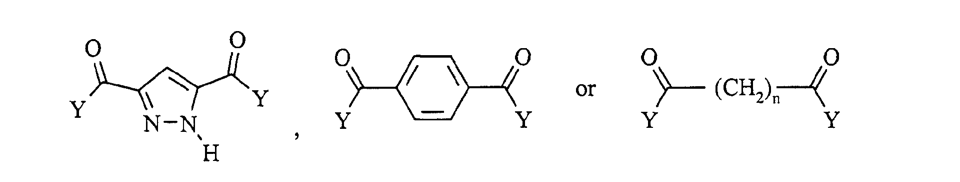 wherein Y is a chlorine atom and n is 2, 3 or 4.

Other suitable heterocyclic compounds of the general formula (4) can be compounds, wherein R² is selected from a substituted or unsubstituted imidazolediyl group, a substituted or unsubstituted oxazole-diyl group, a substituted or unsubstituted isoxazole-diyl group, a substituted or unsubstituted thiazole-diyl group, a substituted or unsubstituted isothiazole-diyl group, a substituted or unsubstituted pyridine-diyl group, a substituted or unsubstituted pyrimidine-diyl group, a substituted or unsubstituted pyridazine-diyl group, a substituted or unsubstituted pyrazine-diyl group, a substituted or unsubstituted pyrrole-diyl group, a substituted or unsubstituted thiophene-diyl group, a substituted or unsubstituted furane-diyl group, a substituted or unsubstituted phenothiazine-diyl group, or a substituted or unsubstituted phenazine-diyl group.

Particularly, R² in the general formula (4) can be selected from a substituted or unsubstituted 1,2-phenylene group, a substituted or unsubstituted 1,3-phenylene group, a substituted or unsubstituted 1,4-phenylene group, a substituted or unsubstituted 1,8-naphthylene group, a substituted or unsubstituted 1,4-naphthylene group, a substituted or unsubstituted 1,5-naphthylene group, a substituted or unsubstituted 9,10-anthracene group, a substituted or unsubstituted 2,2'-biphenylene group, a substituted or unsubstituted 3,3'-biphenylene group, a substituted or unsubstituted 4,4'-biphenylene group, a substituted or unsubstituted 4,4'-stilbene-diyl group, or a substituted or unsubstituted 4,4'-tolane-diyl group.

Moreover, suitable olefinic or acetylenic compounds of the general formula (4) are, for example, maleic acid dichloride, fumaric acid dichloride, acetylene dicarboxylic acid dichloride or derivatives or higher homologues thereof.

In one embodiment of the present invention, the bifunctional compound is represented by the following formula: 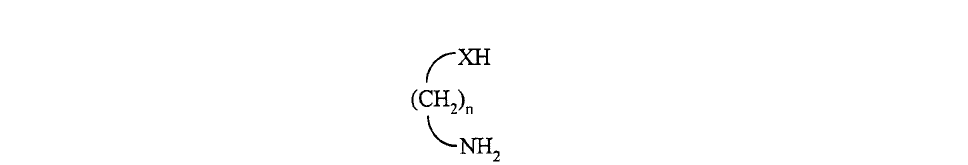 wherein X is as defined above and n is 2, 3 or 4.

According to another embodiment of the present invention, the bifunctional compound used as the starting compound in the process according to the present invention is: 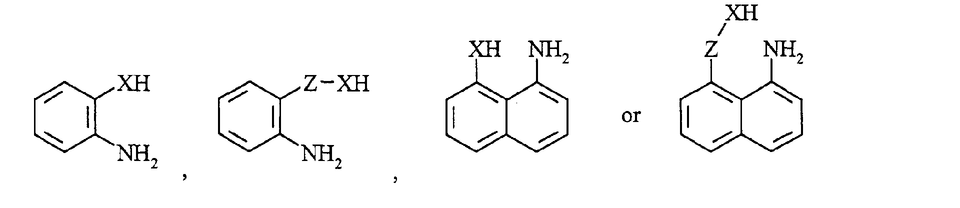 wherein Z is a methylene group, a carbonyl group, a thiocarbonyl group or a C(R³)₂ group with X and R³ being as defined above.

Other suitable bifunctional compounds can be, for example, a substituted or unsubstituted 1,2-diaminocyclohexane, a substituted or unsubstituted 1,2-diaminocyclopentane, a substituted or unsubstituted 1,2-diaminonaphthalene, a substituted or unsubstituted 2,3-diaminonapthalene, a substituted or unsubstituted 9,10-diaminophenanthrene, a substituted or unsubstituted 2,3-diaminopyridine, a substituted or unsubstituted 3,4-diaminopyridine, or a substituted or unsubstituted 4,5-diaminopyrimidine.

According to a further embodiment of the present invention, the bifunctional compound can be: 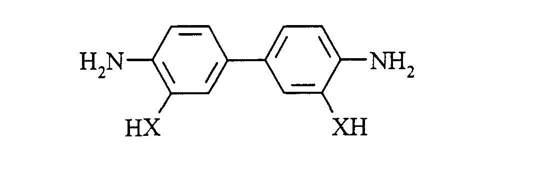 wherein X is O, S,

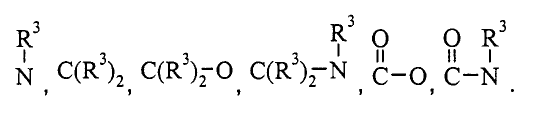

The process according to the present invention is carried out in an aprotic solvent. Suitable aprotic solvents are, for example, acetonitrile, dimethylformamide, dimethylsulfoxide, nitromethane, hexamethylphosphoric triamide, acetone, pyridine, tetrahydrofurane, diethyl ether, benzene, dioxane, trichloromethane or tetrachloromethane, or mixtures thereof. In particular, polar aprotic solvents such as acetonitrile, are preferred.

The process according to the present invention is conducted within a temperature range of from -50°C to 50°C. Preferably, the temperature range is between -20°C and 50°C, more preferably between 0°C and 40°C.

Specific heterocycles which are, for example, obtainable by the process according to the present invention, are represented by the following structures: 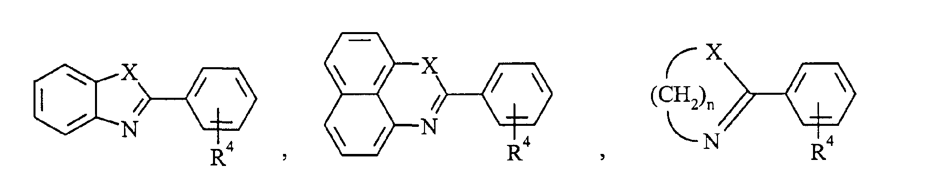 wherein R⁴ is H, OH, N(R³)₂ or NO₂, n is 2, 3 or 4 and with X and R³ being as defined above.

Further preferred heterocycles can be: 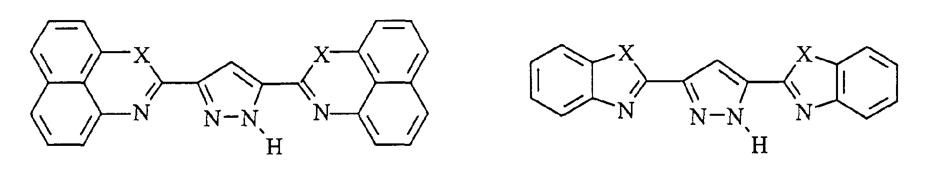 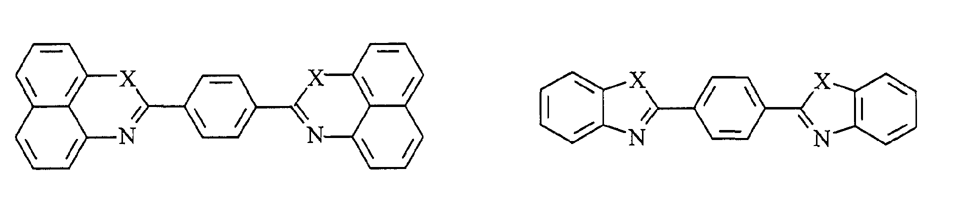 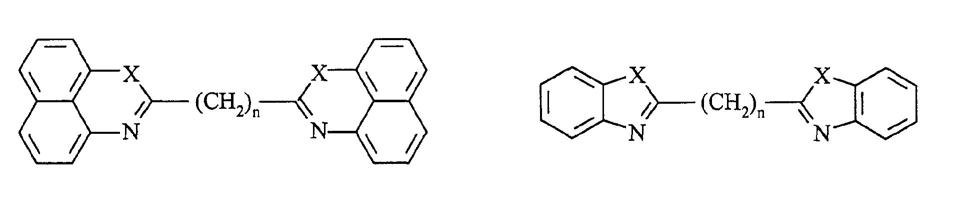 wherein n is 2, 3 or 4 and X being as defined above.

In a further embodiment of the present invention, specific heterocycles obtainable by the process of the present invention can be: 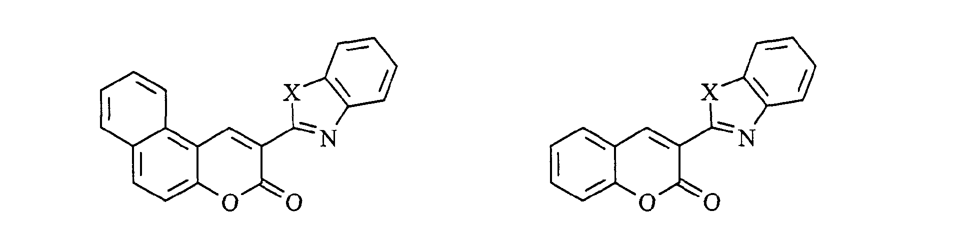 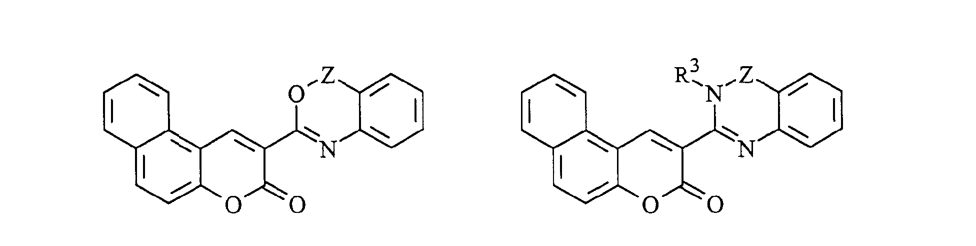 wherein Z is a methylene group, a carbonyl group, a thiocarbonyl group or a C(R³)₂ group with X and R³ being as defined above.

Further preferred heterocycles can be: 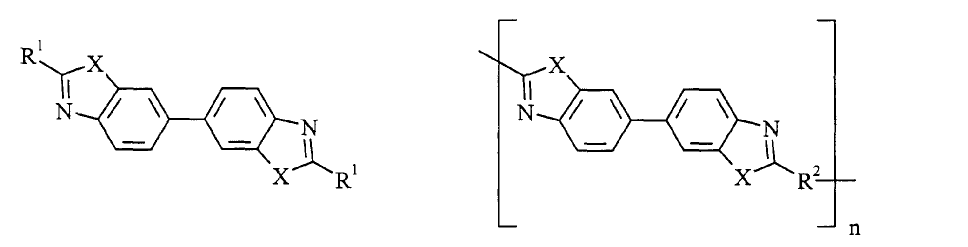 wherein n is an integer of 1 to 50000 and R¹ and R² are as defined above.

The present invention will now be described in further detail with reference to the examples. However, it should be understood that the present invention is by no means restricted to these specific examples.

### Example 1

### General procedure for producing heterocycles having the structural unit (1)

To a stirred solution of 1 mole carboxylic acid chloride (3) dissolved in 200 ml acetonitrile and 2 mole triethylamine, 1 mole of a 2M solution of the bifunctional compound in acetonitrile or N,N-dimethylformamide is added dropwise at room temperature. The reaction temperature is controlled so that it should not exceed 35°C to 40 °C. Stirring is continued for 1 to 5 hours until complete conversion is observed by DC control. Then, the reaction mixture is poured into 2 litre of an aqueous 1M hydrochloric acid solution which is externally cooled with ice. The precipitated product is collected in a Buchner funnel. Generally, the product thus obtained has high purity and can be purified by recrystallization or vacuum sublimation.

### Example 2

### General procedure for producing heterocycles having the structural unit (2)

To a stirred solution of 1 mole dicarboxylic acid dichloride (4) dissolved in 200 ml acetonitrile and 3 mole triethylamine, 2 mole of a 2M solution of the bifunctional compound in acetonitrile or N,N-dimethylformamide is added dropwise at room temperature. The reaction temperature is controlled so that it should not exceed 35°C to 40°C. Stirring is continued for 5 hours to complete the reaction and then, the reaction mixture is poured into 2 litre of an aqueous 1M hydrochloric acid solution which is externally cooled with ice. The crystalline product thus obtained can be purified by recrystallization or sublimation.

## Claims

1. A process for producing heterocycles having at least two heteroatoms at least one of which being a nitrogen atom and having the structural units (1) or (2): 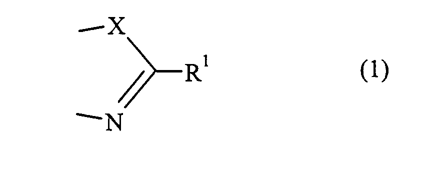 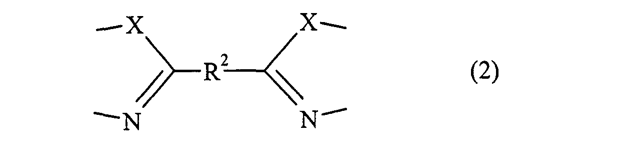 wherein X and N are linked via a substituted or unsubstituted C₂-C₄ alkylene group, via the 1,2-, 1,3- or 1,4-position of a substituted or unsubstituted (C₅-C₇) cycloaliphatic group, via the 1,2-position of the double bond of a substituted or unsubstituted alkenylene group, via the 1,2-position of a substituted or unsubstituted phenylene group wherein the phenylene group can be coupled by a C-C single bond to form a biphenylene group having the structural unit (1) on both phenylic units, via the 1,8-position of a substituted or unsubstituted naphthylene group, or via other substituted or unsubstituted 5- or 6-membered aromatic or cycloaliphatic rings having optionally one or more heteroatoms, or benzofused derivatives thereof,
wherein compounds having the structural unit (2) may be symmetrically or unsymmetrically; and
wherein
X represents O, S, 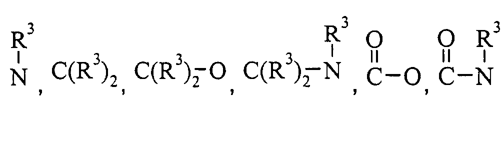 with R³ being each independently selected from hydrogen, a substituted or unsubstituted alkyl group, a substituted or unsubstituted aryl group, or a substituted or unsubstituted heterocyclic group;
R¹ is selected from a carboxyalkyl group, a substituted or unsubstituted alkyl group, a substituted or unsubstituted aryl group, a substituted or unsubstituted heterocyclic group, or derivatives thereof;
R² is selected from a substituted or unsubstituted alkylene group, a substituted or unsubstituted alkenylene group, a substituted or unsubstituted alkynylene group, a substituted or unsubstituted arylene group, or a substituted or unsubstituted bivalent heterocyclic group;
the process comprising the step of reacting a carboxylic acid halogenide having the general formulae (3) or (4): 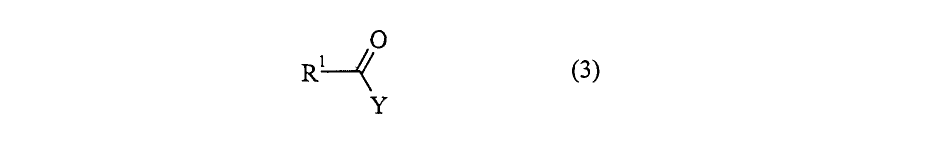 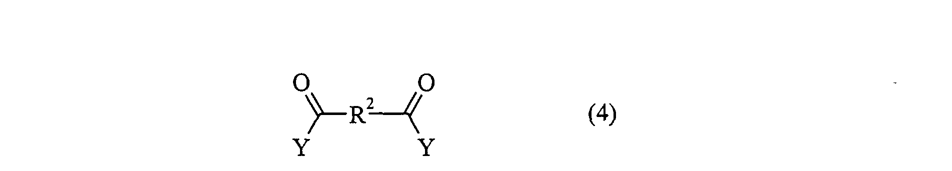 wherein R¹ and R² are as defined above and Y represents a halogen atom, with bifunctional compounds having at least two substituents -XH and -NH₂ at a temperature in the range of -50°C to 50°C in an aprotic solvent, wherein X is as defined above and X and N are linked via a substituted or unsubstituted C₂-C₄ alkylene group, via the 1,2-, 1,3- or 1,4-position of a substituted or unsubstituted cycloaliphatic C₅-C₇ group, via the 1,2-position of the double bond of a substituted or unsubstituted alkenylene group, via the 1,2-position of a substituted or unsubstituted phenylene group wherein the phenylene group can be coupled by a C-C single bond to form a biphenylene group, via the 1,8-position of a substituted or unsubstituted naphthylene group, or via other substituted or unsubstituted 5- or 6-membered aromatic or cycloaliphatic rings having optionally one or more heteroatoms, or benzofused derivatives thereof.

2. The process according to claim 1, wherein Y represents a chlorine atom.

3. The process according to claims 1 or 2, wherein X represents an oxygen atom, a sulphur atom or a group NR³ with R³ being as previously defined.

4. The process according to any one of claims 1 to 3, wherein R¹ in the general formula (3) represents a coumaryl group or benzofused coumaryl group or derivatives thereof, or a substituted aryl or heteroaryl group comprising one or more substituents selected from a hydroxy group, an alkoxy group, an amino group, an alkylamino group, a dialkylamino group, a cyano group, a carboxy group or a nitro group.

5. The process according to any one of claims 1 to 4, wherein compounds of the general formula (4) are: 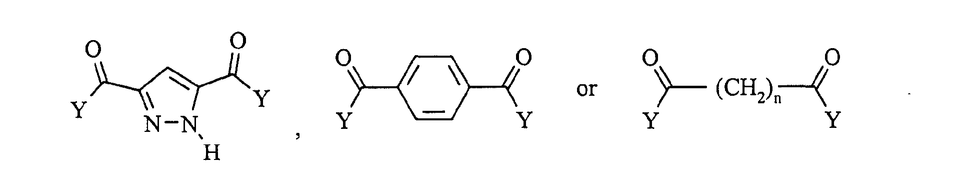 wherein n is 2, 3 or 4 and Y is as previously defined.

6. The process according to any one of the preceding claims, wherein the bifunctional compound has the following structure: 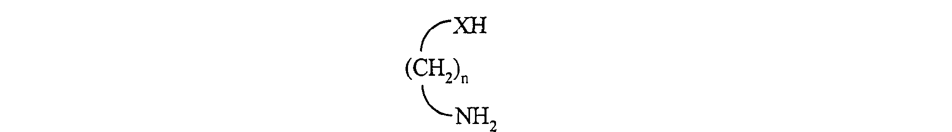 wherein n is 2, 3 or 4 and X is as previously defined.

7. The process according to any one of the preceding claims, wherein the bifunctional compound has one of the following structures: 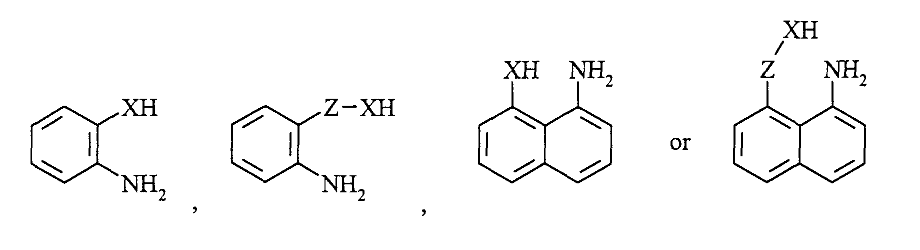 wherein Z is a methylene group, a carbonyl group, a thiocarbonyl group or a C(R³)₂.group with X and R³ being as defined above.

8. The process according to any one of the preceding claims, wherein the bifunctional compound has the following structure: 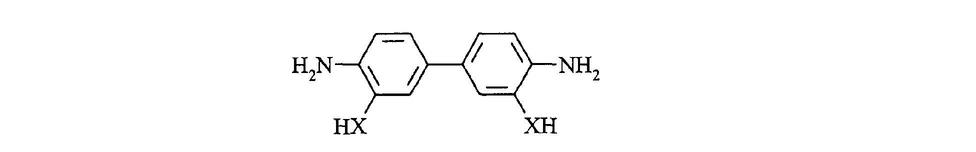 wherein X is O, S,

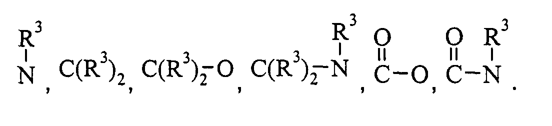

9. The process according to any one of claims 1 to 8, wherein the heterocycles have the following structures: 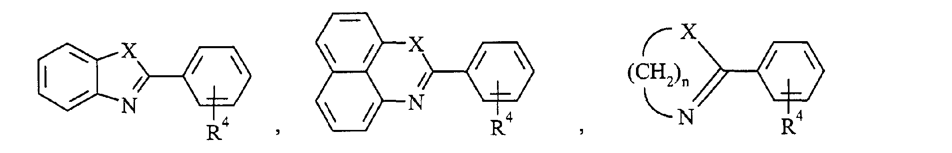 wherein R⁴ is H, OH, N(R³)₂ or NO₂, n is 2, 3 or 4 and X is as previously defined.

10. The process according to any one of claims 1 to 8, wherein the heterocycles have the following structures: 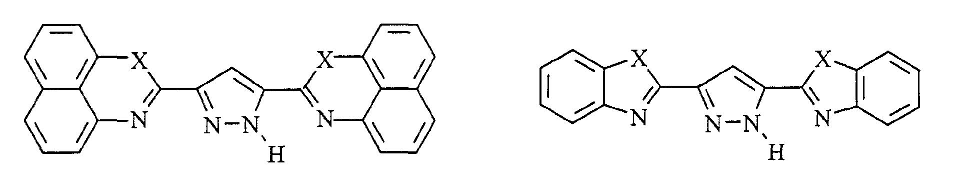 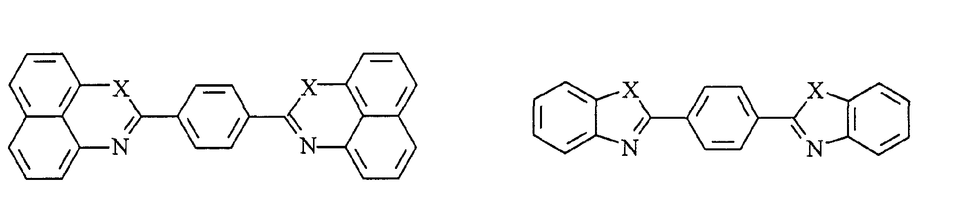 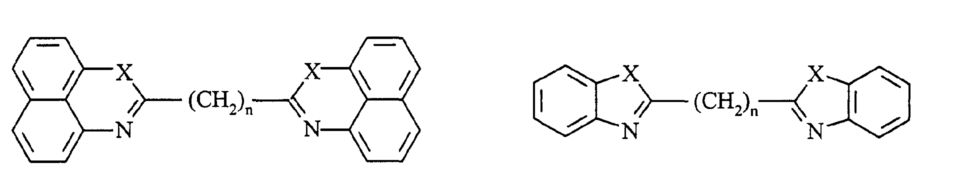 wherein n is 2, 3 or 4 and X is as previously defined.

11. The process according to any one of claims 1 to 8, wherein the heterocycles have the following structures: 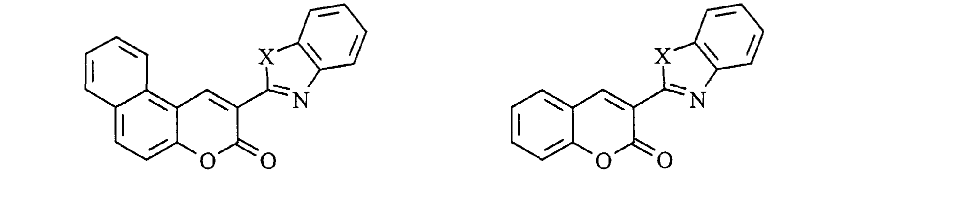 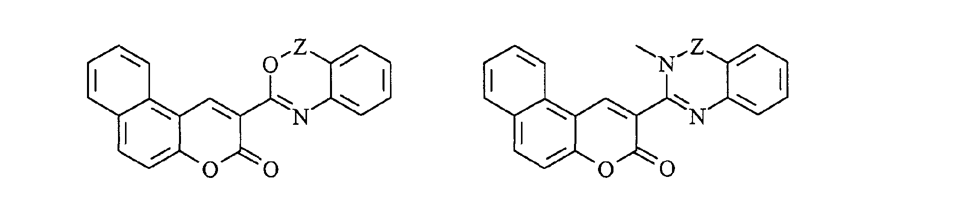 with X and Z being as previously defined.

12. The process according to any one of claims 1 to 8, wherein the heterocycles have the following structures: 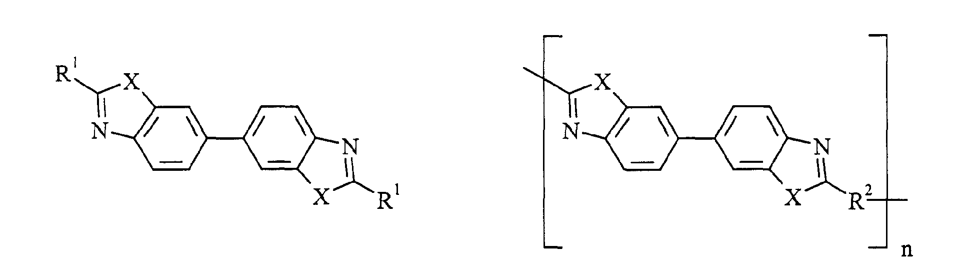 wherein n is an integer of 1 to 50000 and R¹, R² and X are as previously defined.
